# EUROPEAN PATENT APPLICATION

(11) **EP 2 151 452 A1**
(43) Date of publication of application: **10.02.2010**
(21) Application number: 08013552.8
(22) Date of filing: 28.07.2008
(51) Int. Cl.: C07K 14/47

(54) **Bag-1 peptide that inhibits prostate cancer**

(71) Applicant: Karlsruher Institut für Technologie, 76131 Karlsruhe (DE)
(72) Inventor: Cato, Andrew C.B., Dr., 76344 Eggen-Leo (DE); Shatkina, Liubov, 79104 Freiburg (DE); Jehle, Katja, 76185 Karlsruhe (DE); Maddalo, Danilo, 76226 Karlsruhe Durlach (DE)
(74) Representative: Müller-Boré & Partner Patentanwälte

(57) **Abstract**

The present invention relates to a polypeptide having antitumor properties, to a vector encoding said polypeptide, to a host cell containing said vector, to uses of said polypeptide in the manufacture of a medicament for the treatment of cancer in patients, to a method comprising binding said polypeptide to molecular chaperones, and to a pharmaceutical composition containing said polypeptide.

## Description

The present invention relates to a polypeptide having antitumor properties, to a vector encoding said polypeptide, to a host cell containing said vector, to uses of said polypeptide in the manufacture of a medicament for the treatment of cancer in patients, to a method comprising binding said polypeptide to molecular chaperones, and to a pharmaceutical composition containing said polypeptide.

Bag-1 is a family of proteins made up of polypeptides translated from the same mRNA by a leaky scanning mechanism. In humans there are four isoforms of Bag-1: Bag-1L, Bag-1 M, Bag-1S and p29. These proteins function through interaction and regulation of the activities of diverse cellular proteins ranging from Bcl-2, c-Raf, steroid receptors, growth factor rectors, retinoblastoma protein and the molecular chaperone Hsp70/Hsc70. All the Bag-1 proteins have a conserved C-terminal Hsp70 binding domain (otherwise known as Bag-domain) that binds to Hsp70/Hsc70 through which the Bag proteins exert their function as nucleotide exchange factors for Hsp70/Hsc70.

The Bag-domain is also present on other proteins termed Bag-2, Bag-3 (Bis), Bag-4 (Sodd), Bag-5 and Bag-6 (Scythe) that have diverse functions. Several of the Bag proteins have been implicated in the control of apoptosis. Bag-1 and Bag-3 (Bis) interact with Bcl2 to reduce apoptosis induced by several factors. Bag-4 (Sodd) associates with certain receptors of the tumour necrosis factor family and block their signaling. Bag-6 (Scythe) regulates the nuclear pathway that communicates with mitochondria and regulates the release of cytochrome c thereby controlling apoptosis. Structural analysis of the Bag-domains by multi-dimensional nuclear magnetic resonance methods show that the Bag-domain of Bag-1 proteins is structurally and evolutionally distinct from all the other Bag-domain proteins, indicating that the Bag-1 proteins may have properties distinct from the other Bag proteins.

Various isoforms of Bag-1 have been shown to promote a variety of malignant behavior including apoptosis suppression, factor-independent cell growth, increased cell motility and chemoresistance in cell culture experiments. In addition, overexpression of Bag-1 promotes survival of cells in suspension by blocking the phenomenon of anoikis (apoptosis caused by cell detachment from the extracellular matrix).

Increased Bag-1 isoform levels have been reported in different cancers ranging from breast, lung, endometrial, gastrointestinal tract and prostate cancers. Furthermore, Bag-1-isoform overexpression promoted tumor growth and metastatic spread of tumour cells in mouse models and conversely siRNA-mediated Bag-1 expression, Bag-1 gene haploid-insufficiency and expression of dominant-interfering mutants of Bag-1 impair cell survival or interfere with tumorigenesis or progression of cancer. All these demonstrate the relevance of Bag-1 in tumorigenesis.

Further data using Bag-1 heterozygous mice and crossing them with the mouse prostate cancer model TRAMP convincingly shows that Bag-1 is involved in the development of prostate cancer. A reduction of tumor size upon lowering Bag-1 levels in the mice could be shown. A similar result on reduced lung tumor formation was observed when mice with Bag-1 haploid-insufficiency were crossed with mice carrying c-Raf kinase that develop multifocal adenomas early in adulthood. Thus, increased Bag-1 levels are not restricted to prostate cancer formation but are involved in other tumors as previously reported in immunohistological and RNA studies for different tumor types.

While organ-confined tumor can be treated successfully with surgery, advanced or metastatic prostate carcinoma can be handled only by palliative hormonal therapy. The success achieved by this therapy is only temporary as almost all the tumors become refractory to therapy within a few years after the start of treatment. Several mechanisms have been proposed for this resistance.

Among them is an increased expression of coactivators of the androgen receptor in advanced prostate cancer cells. There is therefore a great interest in targeting proteins that bind to the androgen receptor (AR) as therapy for advanced prostate tumor.

The cochaperone Bag-1L has been reported to enhance AR action and to be over-expressed in advanced prostate cancer. Furthermore, increased Bag-1L levels impair the inhibitory effect of the antiandrogen cyproterone acetate and hydroxyflutamide, as well as bicalutamide on transactivation by the AR. These findings make Bag-1L an important protein to target for the treatment of prostate cancer particularly at the hormone refractory stage when the tumor fails to respond to anti-androgen treatment.

The technical problem underlying the present invention is to provide a new polypeptide having antitumor properties, especially against prostate cancer.

The solution to the above technical problem is achieved by the embodiments characterized in the claims.

In particular, the present invention relates to a polypeptide comprising the amino acid sequence having SEQ ID NO. 1 (SEQ ID NO. 1: TPLSALGIQ DGCRVMLIGK KNSPQEEVEL KKLKHLEKSVEKIADQLEEL NKELTGIQQG FLPKDLQAE) or to a functionally active derivative thereof, wherein the poylpeptide is not naturally occurring Bag-1.

Naturally occurring Bag-1 according to the present invention can be any isoform of Bag-1, preferably an isoform selected from the group consisting of Bag-1L, Bag-1 M, Bag-1S and p29.

In a preferred embodiment, the present invention relates to a polypeptide consisting of amino acids 202-269 of the Bag-1L protein or to a functionally active derivative thereof. Accordingly, in a preferred embodiment, the present invention relates to a polypeptide consisting of SEQ ID NO. 1 or to a functionally active derivative thereof.

The term "functionally active derivative thereof" as used herein relates to derivatives which contain deletions, additions and/or substitutions of amino acids, the presence, absence or substitution of which does not have a substantial influence on the activity of the polypeptide, e.g. conservative amino acid substitutions, i.e. substitution of an amino acid by an amino acid having similar chemical properties. The term "derivative" includes post-translational modifications, e.g. glycosylation patterns that differ from the wild-type.

In a preferred embodiment of the present invention, a functionally active derivative of the polypeptide according to the present invention comprises an amino acid sequence having 70%, more preferred 80%, more preferred 90%, more preferred 95%, and even more preferred 98% identity with SEQ ID NO. 1 which has essentially the same activity as a polypeptide comprising the amino acid sequence having SEQ ID NO. 1.

In a preferred embodiment of the present invention, the polypeptide is produced synthetically, means for which are well known in the art.

In another preferred embodiment of the present invention, the polypeptide is produced recombinantly. The recombinant polypeptide of the present invention may be produced by any method known in the art. This may include any method known in the art for (i) the production of recombinant DNA by genetic engineering, e.g. via reverse transcription of RNA and/or amplification of DNA, (ii) introducing recombinant DNA into prokaryotic or eukaryotic cells by transfection, e.g. via electroporation or microinjection, (iii) cultivating said transformed cells, e.g. in a continuous or batchwise manner, (iv) expressing the polypeptide of the present invention, e.g. constitutively or upon induction, and (v) isolating said polypeptide, e.g. from the culture medium or by harvesting the transformed cells, in order to (vi) obtain purified recombinant polypeptide, e.g. via anion exchange chromatography or affinity chromatography.

The polypeptide according to the present invention can contain regions that facilitate the purification of a recombinantly produced polypeptide, for example detectable amino acid sequences well known in the art, e.g. a His- or Flag-Tag at the C- or N-terminus.

One aspect of the present invention relates to the use of a polypeptide of the present invention to bind to at least one molecular chaperone. Preferred molecular chaperones are GRP75 and/or GRP78. Accordingly, one aspect of the present invention is a method for the detection of a cell, preferably a neoplastic or cancer cell, expressing a molecular chaperone, preferably GRP75 and/or GRP78, said method comprising (a) contacting said cells with a polypeptide of the present invention under conditions suitable for the binding of said polypeptide to the molecular chaperone, and (b) detecting said polypeptide.

The polypeptide of the present invention can be detected qualitatively and/or quantitatively by methods well known in the art. Examples for the detection of the above polypeptide are, but not limited to, the use of a labelled antibody directed against the above polypeptide. Further, according to the present invention, the polypeptide can be linked, preferably covalently linked, to at least one detectable label which may be any suitable detectable label known in the art. The term "detectable label" does not exhibit any particular limitation and may be selected from the group consisting of radioactive labels, fluorescent dyes, compounds having an enzymatic activity, magnetic labels, antigens, and compounds having a high binding affinity for a detectable label. The detection method for measuring the detectable label can be, for example, selected from the group consisting of an enzyme assay, a chromogenic assay, a lumino assay, a fluorogenic assay, and a radioimmune assay. The reaction conditions to perform detection of the detectable label depend upon the detection method selected. It is within the knowledge of a person skilled in the art to choose the optimal parameters, such as buffer system, temperature and pH for the respective detection system used.

In another aspect, the present invention relates to a nucleic acid comprising a nucleotide sequence encoding a polypeptide of the present invention.

In a preferred embodiment, the nucleotide sequence encoding a polypeptide of the present invention is SEQ ID NO. 2 (SEQ ID NO. 2: 5'-ACACCGTTGTCAGCACTTGGAATACAAGATGGTTGCCGGGTCATGTTAATT GGGAAAAAGAACAGTCCACAGGAAGAGGTTGAACTAAAGAAGTTGAAACA TTTGGAGAAGTCTGTGGAGAAGATAGCTGACCAGCTGGAAGAGTTGAATA AAGAGCTTACTGGAATCCAGCAGGGTTTTCTGCCCAAGGATTTGCAAGCT GAA-3').

The term "nucleic acid" as used herein relates to a native, semisynthetic, synthetic or modified nucleic acid molecule consisting of desoxyribonucleotides and/or ribonucleotides and/or modified nucleotides.

In another aspect, the present invention relates to a vector containing the above-mentioned nucleic acid. The vector of the present invention is not subjected to any particular limitations, whereas suitable vectors are known in the art. In a preferred embodiment, the vector of the present invention is capable of expressing the polypeptide according to the present invention in a prokaryotic and/or eukaryotic host cell. For this purpose, the vector can contain suitable regulatory elements, e.g. promoters, enhancers and termination sequences. Further, the vector can be used for the stable integration of the nucleic acid according to the present invention into the genetic material of a host cell.

Accordingly, another aspect of the present invention relates to a host cell that contains the above-mentioned nucleic acid and/or the above-mentioned vector. Suitable host cells are bacterial cells, yeast cells, plant cells, insect cells, and mammalian cells. Especially preferred are *E*. *coli* cells or insect cells.

Moreover, one aspect of the present invention relates to a method for the production of the polypeptides of the present invention. This method comprises (a) culturing the host cell of the present invention in a suitable culture medium under conditions that are conducive for the expression of said polypeptide, and (b) isolating said polypeptide from the host cell and/or the culture medium.

The polypeptide according to the present invention is preferably expressed with the help of suitable expression systems well known in the art, preferably as a secreted product. Preferred expression systems for the recombinant expression are E.coli and insect cells.

A further aspect of the present invention relates to the use of a polypeptide of the present invention for the induction of apoptosis in a cell. In a preferred embodiment, apoptosis is induced *in vivo*, e.g. in neoplastic tissue or a tumor of a patient. In another preferred embodiment, apoptosis is induced *in vitro,* e.g. in a cell culture. Accordingly, one aspect of the present invention relates to a method for the induction of apoptosis in a cell culture, comprising contacting said cell culture with a polypeptide of the present invention.

Due to the antitumor properties of the polypeptide of the present invention, one aspect of the present invention relates to the polypeptide, nucleic acid and vector of the present invention for use as a medicament.

In one aspect, the present invention relates to the polypeptide of the present invention for use in the treatment of a neoplastic condition in a patient.

In another aspect, the present invention relates to the use of a polypeptide of the present invention in the manufacture of a medicament for the treatment of a neoplastic condition in a patient.

In a preferred embodiment, the neoplastic condition is a condition associated with a solid tumor. In a more preferred embodiment of the present invention, the neoplastic condition is selected from the group consisting of breast cancer, lung cancer, endometrial cancer, gastrointestinal tract cancer and prostate cancer. In an especially preferred embodiment, the neoplastic condition is prostate cancer. In an even more preferred embodiment, the prostate cancer is a hormone refractory prostate cancer. In further preferred embodiments, the patient is a human.

The term "neoplastic condition" as used herein is known to a person skilled in the art and may relate to any abnormal mass of tissue, the growth of which exceeds and is uncoordinated with that of the normal tissues, and persists in the same excessive manner after cessation of the stimulus which evoked the change.

In another preferred embodiment, the neoplastic condition according to the present invention is a solid tumor. In a further preferred embodiment, the neoplastic condition is a transformed cell.

In another aspect, the present invention relates to the use of a nucleic acid of the present invention and/or a vector of the present invention in the manufacture of a medicament for the treatment of a neoplastic condition in a patient.

Accordingly, in another aspect, the present invention relates to a pharmaceutical composition comprising, in a therapeutically effective amount, a polypeptide of the present invention and/or a nucleic acid of the present invention and/or a vector of the present invention, optionally in combination with one or more pharmaceutically acceptable excipient(s) and/or carrier(s) and/or diluent(s) and/or solvent(s) and/or salt(s) and/or buffer(s). The pharmaceutical composition of the present invention can have any suitable form that is known in the art. Preferably, it is a solid form, a liquid form or in form of an aerosol.

In a preferred embodiment, the pharmaceutical composition of the present invention is for the treatment of a neoplastic condition in a patient.

In one aspect, the present invention relates to a method for the treatment of a neoplastic condition in a patient, said method comprising administering to a patient a polypeptide and/or a nucleic acid and/or a vector and/or a pharmaceutical composition according to the present invention. Such administration is not subjected to any particular limitations and can be in any suitable form known in the art, e.g. orally, intravenously, intradermal, intraperitoneal, intramuscular, or directly into the neoplastic tissue or tumor. In a preferred embodiment, the neoplastic condition is hormone refractory prostate cancer, and the treatment is conducted in the absence of any anti-androgen treatment.

In one other aspect, the present invention relates to a method for producing the pharmaceutical composition of the present invention, comprising mixing a polypeptide of the present invention and/or a nucleic acid of the present invention and/or a vector of the present invention with one or more pharmaceutically acceptable excipient(s) and/or carrier(s) and/or diluent(s) and/or solvent(s) and/or salt(s) and/or buffer(s).

The present invention further relates to a method for the detection of a cell expressing at least one molecular chaperone, comprising
(a) contacting said cell with the polypeptide according to the present invention under conditions suitable for the binding of said polypeptide to the molecular chaperone; and
(b) detecting said polypeptide.

In a preferred embodiment of the present invention the molecular chaperone is selected from the group consisting of GRP75 and GRP78.

In another preferred embodiment of the present invention the cell expressing at least one molecular chaperone is a neoplastic or a cancer cell.

The figures show:

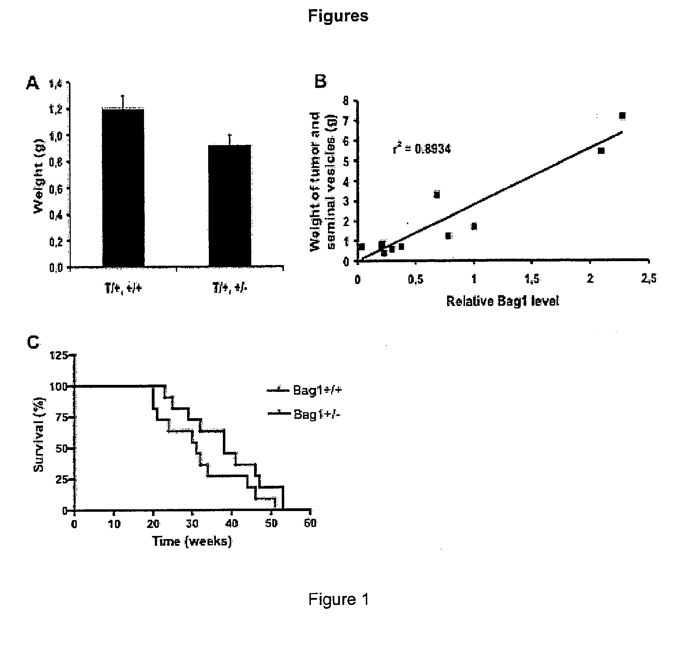

Figure 1: Bag-1 haploinsufficiency impairs prostate cancer development in a TRAMP mouse model. Bag-1 heterozygous mice were crossed with TRAMP mice and progeny were analyzed for the weight of the prostate tumor and its correlation with Bag-1 levels as was the overall survival of the mice. (A) Weight of the prostate and seminal vesicles of TRAMP/Bag-1+/+ mice (n=17) and TRAMP/Bag-1+/- mice (n=16) at 25 weeks. (B) Weight of prostate tumor and seminal vesicles correlated with Bag-1 levels. Bag-1 protein level (p32) was determined by immunoblot analysis and expressed relative to the level of a-actin and compared with the weight of the prostate tumor and seminal vesicles (n=12). (C) Kaplan-Meier curve showing the survival rate of TRAMP/Bag-1 +/+ mice (light gray) compared with TRAMP/Bag-1+/- mice (p=0.16).

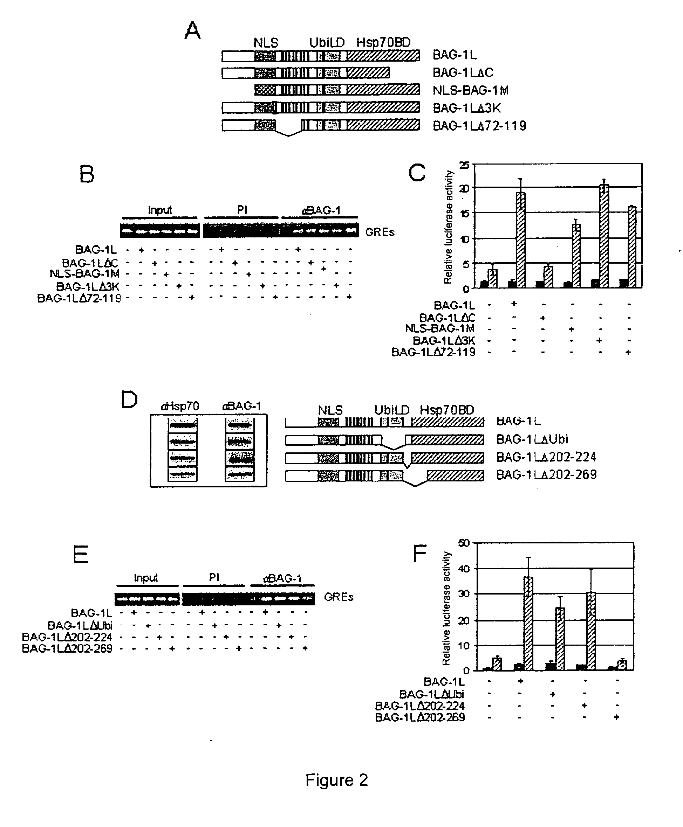

Figure 2: The region between amino acids 202 and 269 of Bag-1L is essential for DNA binding and BAG-1L-mediated enhancement of transactivation by the AR. (A, D) Schematic presentation of Bag-1L and its deletion mutants. (B, E) Cos7 cells were transfected with a reporter MMTV LTR-containing plasmid together with empty vector, wt Bag-1L or its deletion mutants. 40h after transfection, cells were fixed with 1% formaldehyde for 20 min, harvested and subjected to chromatin immunoprecipitation. The immunoprecipitated DNA fragments were analyzed by PCR. The pictures show a representative experiment of 3 independent experiments. (C, F) Cos7 cells were transfected with a MMTV Luciferase reporter construct together with an AR-encoding plasmid and an empty expression vector, wt Bag-1L or its deletion mutants. In addition, a *Renilla* luciferase construct was co-transfected as an internal control for the efficiency of transfection. 24h after transfection, cells were stimulated either with 10⁻⁷ M DHT or vehicle for 16 h, harvested and subjected to luciferase assay. The bar charts show the average relative luciferase activity of 4 independent experiments with standard deviations.

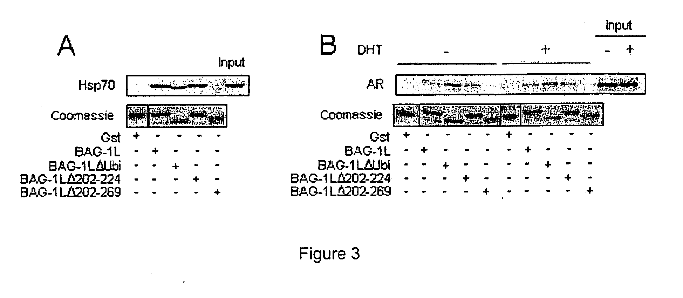

Figure 3: The region between amino acids 202 and 269 of Bag-1L is essential for binding to Hsp70 and AR. 300µg of the protein from PC3 whole cell extract (A) or *in vitro* translated [³⁵S]-methionine-labeled human AR (B) were incubated with glutathione-agarose beads to which GST, GST-Bag-1L, GST-Bag-1LΔUbi, GST-Bag-1LΔ202-224 or GST-Bag-1LΔ202-269 were bound. *In vitro* translated AR was incubated with or without 10⁻⁷M DHT before use in the binding reaction. The beads were washed and bound proteins were separated by SDS-PAGE and detected by immunoblotting with the antibody against Hsp70 (A, upper panel) or autoradiography (B, upper panel). 5% of the proteins used in the binding reactions were loaded onto the gel as input. To control the amount of the GST-tagged proteins the membrane was stained with Ponceau S (A, lower panel) or the gel was stained with Coomassie blue (B, lower panel).

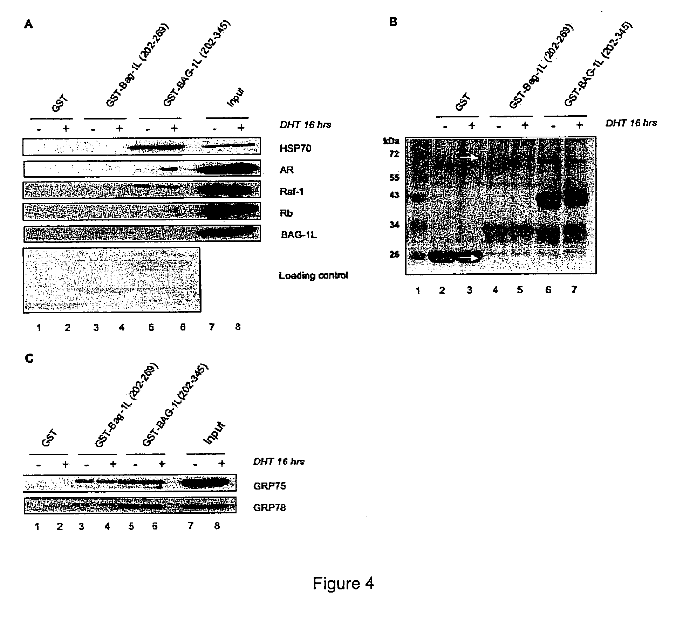

Figure 4: Identification of proteins interacting with the Bag-1L (202-269) peptide. GST-pull down studies were carried out with 15 µg GST-Bag-1L-(202-269), GST-Bag-1L-(202-345) or GST alone as control and 400 µg total cell lysate from 22Rv.1 cells treated with vehicle alone or with 10⁻⁷ M DHT for 16h. The bound material was analyzed on 10% SDS-PAGE and immunoblotting was carried out using different antibodies. (A) After separation of the bound material, immunoblot was carried out using antibodies directed against Hsp70, AR, Raf-1, Rb and human Bag-1L. A coomassie stained gel is also shown as control of the amount of the GST proteins loaded on the gel. (B) After the pull-down experiment and separation of the bound material, the gel was silver stained to identify the proteins that bind to the Bag-1L peptide. (C) After separation of the bound material, immunoblot was carried out using antibodies directed against GRP75 and GRP78.

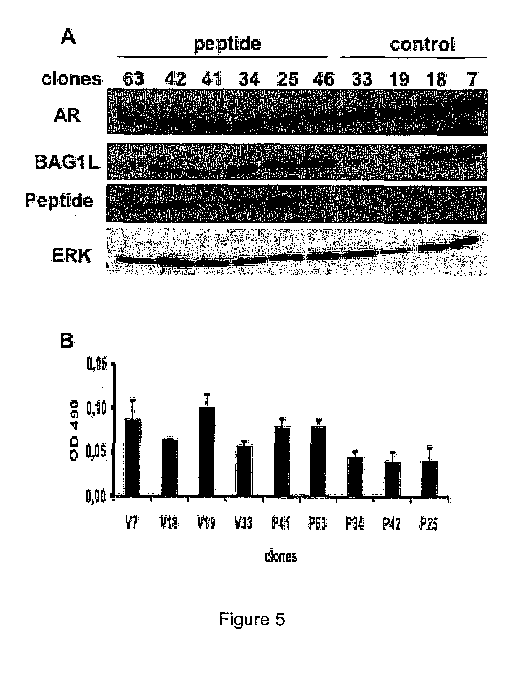

Figure 5: Bag-1L blocking peptide overexpression reduces proliferation in vitro. (A) Cell extracts of 22Rv.1 stable cell lines were analyzed by 10-15% SDS-PAGE and immunoblot using antibodies against AR, Bag-1-L and HA. Staining for ERK1/2 was used as loading control. Stable clones overexpressing the peptide are P34, P41, P46, P63, P25 and P42. The clones with empty expression vectors are 7, 18, 19 and 33. (B) BrdU proliferation assay of stable clones expressing different levels of Bag-1 blocking or empty expression vectors. DNA synthesis was assessed after 24h. This is the average and standard deviation of 3 independent assays.

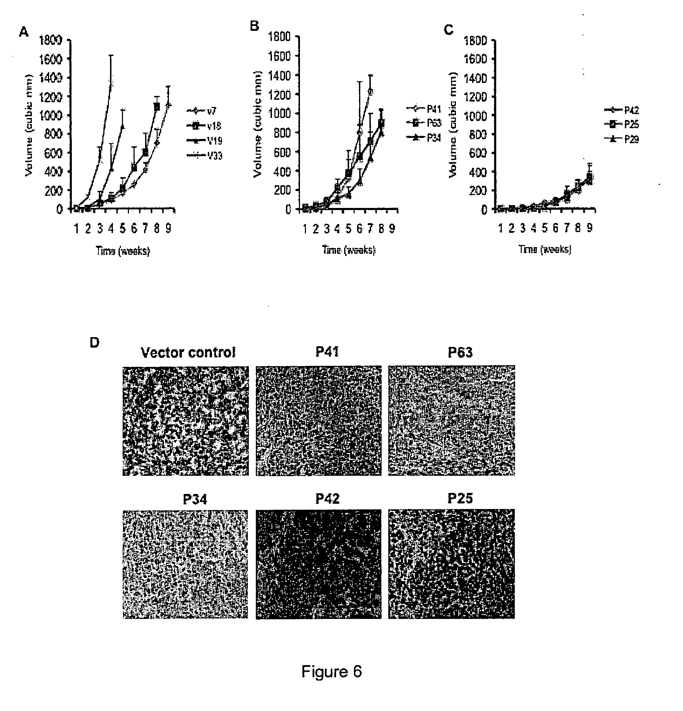

Figure 6: Bag-1L peptide inhibits tumor growth in vivo. Athymic nude mice were injected in both flanks subcutaneously with 22Rv.1 clones. Tumor size was measured once per week using a caliper and expressed as tumor volume in mm³. Shown are the tumor volumes of clones transfected with empty expression vector (A), clones expressing low levels of the Bag-1L blocking peptide (B) and clones expressing high levels of the peptide (C). Each point represents the mean volume and standard deviation of at least four to 10 tumors. (D) Determination of apoptotic levels in tumor tissues. Five-micron sections of formalin-fixed, paraffin-embedded tumor tissues were stained for apoptotic cells using the TUNEL assay. Shown are representative images of the sections of the different tumors.

The present invention will now be further illustrated in the following examples without being limited thereto.

### Examples

### Experimental procedures:

*Cell culture*. Human prostate carcinoma PC3 and monkey kidney COS-7 cell lines were cultured in Dulbecco's modified Eagle's medium while 22Rv.1 cells and LNCaP cells were cultured in RPMI 1640 medium. All culture media were supplemented with 10% fetal bovine serum and the cells were kept at 37°C in an atmosphere of 5% CO₂.

*Antibodies*. Rabbit polyclonal antibody to Bag-1L has already been described. Rabbit monoclonal antibody against GRP75 (H155), goat monoclonal antibodies GRP78 (N20), mouse monoclonal antibody against a-Tubulin (TU-02), rabbit polyclonal antibodies against Bag-1 (C-16) and goat polyclonal antibodies against Hsp70 (K-20) were purchased from Santa Cruz Biotechnology, Heidelberg, Germany. Anti-Rb (Ab-6) mouse monoclonal antibody (AF11) was obtained from Calbiochem. Phospho-Rb (Ser780) antibodies and rabbit polyclonal c-Raf antibody were from Cell Signaling Technology.

*Animals.* C57/BI6 (Bag-1+/-) mice were bred and crossed in house. All experiments were performed according to European and German statutory regulations. The genotype of the mice was established by tissue biopsies and subsequent DNA analysis by PCR.

*Tumor xenograft studies*. Six-week-old male athymic (nude) mice were injected with 5x10⁶ cells 22Rv.1 stably transfected with the Bag-1 peptide (pcDNA3.HA.NLS Bag-1L(202-269)) or empty expression vector suspended in phosphate buffered saline. Each animal received two subcutaneous injections of tumor cells, one on each flank. Primary tumor growth was assessed by measuring the external volume of each tumor once every 7 days using calipers measuring three mutually perpendicular diameters (mm). The geometric mean diameter was then used to calculate the tumor volume in mm³ using the equation V=(1/6) [π] (d1d2d3). At the end of the experiment animals were sacrificed and tumors were weighed. Samples were then collected for immunoblot and for immunohistochemical analysis.

*Expression vectors and plasmids.* The plasmids pGL3MMTV, pTK Renilla Luc and constructs encoding Bag-1L, Bag-1LΔC and NLS-Bag-1M, as well as pGex-4T-1-Bag-1L have been previously described. The MMTV-LTR Cat9 construct was described previously. Constructs encoding Bag-1LΔ3K, Bag-1LΔUbi, Bag-1LΔ202-224 and Bag-1LΔ202-269 were created by replacement of a SacII-XbaI fragment of the Bag-1L cDNA in pcDNA3-Bag-1L with a PCR generated fragment in which the sequence encoding amino acids 73 to 75, 161 to 203, 202 to 224 or 202 to 269, respectively, had been deleted. pcDNA3-Bag-1LΔ72-119 was generated by replacement of a SacII-PflMI fragment of Bag-1L cDNA with the PCR obtained sequence with deleted amino acids 72 to 119. The Bag-1L blocking peptide was created by PCR amplification where an HA sequence followed by the nuclear localization sequence (NLS) of simian virus 40 in frame with amino acid sequences 202-269 from Bag-1L to generate pcDNA3.HA.NLS Bag-1L(202-269). As a control, the HA sequence was introduced into pcDNA3 vector to generate the empty expression vector pcDNA3.HA. For GST-pull-down experiments pGex4T.1-Bag-1L (202-269), pGex4T.1-Bag 1L (202-345) by PCR, pGex-4T-1 Bag-1LΔ202-269, pGex-4T-1 Bag-1LΔ202-224 and pGex-4T-1 Bag-1LΔUbi were created by substituting a SacII-BglII fragment of Bag-1L cDNA within pGex-4T-1-Bag-1L with the corresponding fragment from pcDNA3-Bag-1LΔ202-269, pcDNA3-Bag-1LΔ202-224 or pcDNA3-Bag-1LΔUbi, respectively.

*GST pull-down experiments.* Expression of GST fusion proteins and GST pull-down experiments were performed essentially as described previously except that the cells were treated with 10⁻⁷ M dihydrotestosterone (DHT) or vehicle alone for 16h. Cells were then harvested, lysed in 10mM phosphate buffer pH 7.5, 1 mM EDTA, 60mM KCI, 15% glycerol and disrupted twice by sonification (5 sec each) before use. 15 µg of GST-fused protein in phosphate buffered saline (PBS), 10% glycerol, 1 mM DTT were mixed with glutathione-sepharose 4B at room temperature for 30 min, washed two times with 1x PBS (-MgCl₂, -CaCl₂) (GIBCO-BRL, Invitrogen GmbH, Karlsruhe, Germany) and once with LBST (20mM HEPES-KOH, pH 7.9, 60mM NaCl, 2,5mM MgCl₂, 0.1 mM EDTA, 0.05% NP-40, 1.5% Triton X-100). The beads were thereafter incubated with approximately 400 µg proteins of the 22 Rv.1 cell lysate in LBST with 1mM PMSF and 1mM DTT. Lysates were then incubated with the GST-fused proteins 30 min at room temperature and 30 min at 4°C. The beads were washed 4 times with LBST and resuspended in SDS sample buffer and subjected to SDS polyacrylamide gel electrophoresis. Pull-down experiments with GST-fused Bag-1L and its deletion mutants were carried out either with the [S³⁵]-labeled *in vitro* translated AR in the presence or absence of 10⁻⁷ M DHT, or with 300 µg protein from PC3 whole cell lysate. The bound proteins were subjected to SDS-PAGE and autoradiography or western blot analysis with probing with antibody against Hsp70, respectively.

*Cell transfection.* Transient transfection was performed with FuGENE 6 transfection reagent (Roche Diagnostics) with 2x10⁵ PC3 cells in 3.5-cm-diameter dishes. The cells were cultured in Dulbecco's modified Eagle's medium supplemented with 3% charcoal-treated fetal calf serum (CCS) for 20h prior to transfection. Unless otherwise stated, all transfection experiments were performed with luciferase firefly indicator genes and a *Renilla* luciferase construct as an internal control for the efficiency of transfection. The cells were harvested 36h to 44h after transfection in passive lysis buffer (Promega, Mannheim, Germany) for luciferase activity measurements. Stable transfection was carried out with 22Rv.1 cells with 10 µg of plasmid DNA (pcDNA3.HA.NLS, Bag-1L(202-269) or pcDNA3.HA) using FuGene^{™}. Stably transfected cells were selected with 0.8 mg/ml G418.

*Chromatin immunoprecipitation*. Chromatin immunoprecipitation experiments were carried out essentially as described, except that the experiments were carried out in Cos-7 cells transiently transfected with a MMTV-LTR Cat 9 reporter construct and Bag-1L and its deletion mutant expression constructs. Primers for the ChIP were MMTV for (5'-CTGCAGATACTTTTGGCATGAGC-3', SEQ ID NO. 3) and MMTV rev (5'-GGTTTACATAAGCATTTACATAAGACTTGG-3', SEQ ID NO. 4).

*Immunoblotting.* Immunoblot analyses was performed as described previously using cell or tissue extracts. For the preparation of cell lysate, cells were washed once with ice-cold PBS 1x, scraped and centrifuged at 4000g, 5 min at 4°C. The pellet was resuspended in lysis buffer containing 150 mM NaCl, 50 mM Tris pH 8.0, 5 mM EDTA, 1% NP40 and 1:100 protease inhibitor (SIGMA). The extract was further dispersed by sonification and the protein content quantified by the Bio-Rad protein assay (Bio-Rad). Frozen tissues were homogenized in ice-cold lysis buffer containing 1mM phenylmethanesulphonyl-fluoride (PMSF) in a potter homogenizer. The extracts were further dispersed by sonification. Protein lysate samples were subjected to SDS-PAGE on a 10-15% polyacrylamide gel. Immunoblot was carried out using the following antibodies: mouse monoclonal AR antibody from BioGenex (clone F39.4.1), rabbit antiserum raise against amino acids 543 to 558 from the AR, rabbit polyclonal antibody to Bag-1L has been described previously.

*Immunohistochemistry*. Tissues were fixed in 10% formalin for 16h at room temperature, stored in ethanol (50%), paraffin embedded and sectioned 5 µm thick with a Leica RM 2155 microtome. Tissue sections were deparaffinized in xylene and rehydrated in serial alcohol dilutions. Staining was performed according to a streptavidin-biotin-peroxidase protocol. Apoptotic cells were detected via terminal desoxynucleotidyl-transferase-mediated desoxyuridine-triphosphate-biotin nick end labeling (TUNEL) assay using the DNA fragmentation ApopTag *in situ* apoptosis detection kit (Millipore). Briefly, paraffin-embedded tissue was deparaffinized with xylene, digested with proteinase K (20 µg/ml) for 15 min at room temperature, and quenched in 3% hydrogen peroxide for 5 min. After labeling for 1 hour at 37°C, slides were incubated with antidigoxigenin conjugate and color was developed in peroxidase substrate. Slides were counterstained with hematoxilin and analyzed by transmission light microscopy. Samples incubated with a nucleotide mixture lacking terminal transferase were used as a negative control.

*Proliferation assay.* 22Rv.1 stable clones were assayed for proliferation using the kit Cell Proliferation ELISA, BrdU (Roche). Briefly, cells were seeded into a 96 well plate in RPMI 1640 + 10% FCS. The next day BrdU was added to the medium to a final concentration of 10 µM. After 24h cells were harvested, fixed and DNA denatured. Thereafter, an anti-BrdU POD antibody was applied for 2h. Cells were then washed and the immune complexes were detected by subsequent substrate reaction. The reaction product was quantified using a scanning multiwell spectrophotometer (ELISA reader). The developed color and thereby the absorbance values directly correlate to the amount of DNA synthesis and to proliferating cells in the respective microculture.

### Example 1:

### Haploid-insufficient Bag-1 inhibits the development of prostate cancer

To determine the extent of involvement of Bag-1 in prostate cancer development, mice from the transgenic adenocarcinoma of the mouse prostate (TRAMP) system were used and crossed with Bag-1 haploid-insufficient mice to generate Bag-1 +/-; T+ and Bag-1 +/+; T+ mice. The transgenic adenocarcinoma of the mouse prostate (TRAMP) system was generated in C57/BL6 mice by the expression of the SV40 early genes (T- and t-antigens, Tag) under the control of the rat probasin (PB) promoter. Expression of the PB-Tag is detected as early as 4 weeks of age and the earliest pathology is prostatic intraepithelial hyperplasia (PIN) that is detected at 12 weeks of age. By about 18 weeks, the mice commonly display a progressive form of adenocarcinoma and will ultimately develop poorly differentiated carcinoma by the time they reach 24 to 30 weeks of age. By crossing these transgenic mice with the Bag-1 knock-out mice it was possible to determine whether Bag-1 had any effect on the development of prostate cancer. As Bag-1 knockout mice die at embryonic day 12.5-13.5 due to massive apoptosis in the liver and nervous system, it was only possible to use heterozygous Bag-1 mice in our studies.

The progeny of the TRAMP/Bag-1 crosses were sacrificed at 25 weeks of age and the prostate and seminal vesicles were removed and weighed since the tumors in the prostate extended to the seminal vesicles in some mice. The Bag-1+/-/TRAMP tumors were 23% reduced in size (p = 0.033) compared to the Bag-1+/+/TRAMP tumors (Fig. 1A). A similar difference was observed when the mice received testosterone injections. Thus, the effect of Bag-1 appears to be independent of the addition of androgens. The weight of the tumors correlated well with the level of Bag-1 as determined by immunoblot analysis (Fig. 1 B). Furthermore Kaplan-Maier survival analysis showed that reduced Bag-1 levels in the TRAMP/Bag-1 +/- mice were associated with longer survival time compared to the TRAMP/Bag-1+/+ mice, although this difference is not significant (p = 0.15). These studies together demonstrate that Bag-1 levels are important for the progression of prostate cancer.

### Example 2:

### Bag-1L is bound to chromatin

In humans, the Bag-1 gene codes for four proteins: Bag-1L (p50), Bag-1M (p46), Bag-1S (p36) and p29, translated from the same mRNA. So far, increased Bag-1L protein levels have been associated with hormone refractory prostate cancer and implicated in the failure of anti-androgen therapy. Therefore, it was decided to analyze more closely the role of Bag-1L in androgen receptor action in human prostate cancer progression.

It has previously been shown in chromatin immunoprecipitation (ChIP) experiments in the human prostate cancer cell line 22Rv.1 that Bag-1L binds to the androgen response element (ARE) of the prostate specific antigen (PSA) gene even in the absence of androgen. However, in the presence of hormone the Bag-1L binding is decreased and the AR interacts strongly with its response elements on the PSA promoter, implicating Bag-1L in the regulation of androgen receptor action in prostate cancer cells.

To analyze more carefully the significance of chromatin binding to the AR response, ChIP experiments were carried out to determine the region of Bag-1L that binds to chromatin at the hormone response element of the MMTV LTR and to find out whether it also inhibits AR response. The MMTV LTR reporter plasmid and different deletion constructs of Bag-1L were cotransfected into Cos-7 cells for ChIP and reporter gene assays. A construct lacking the last 37 carboxy-terminal amino acids (Bag-1LΔC), a construct lacking the N-terminal sequence but containing the nuclear localization sequence of SV40 that turns it into a nuclear protein (NLS-Bag-1M), a Bag-1L construct with mutation of three lysine residues at positions 73-75 reported to destroy binding of Bag-1L to nucleoli and a Bag-1L mutant lacking the [EEX₄]ₙ repeat motif in the protein (Fig. 2A) were used. In ChIP experiments the proteins coded by all these genes bound chromatin and in transfection experiments they all enhanced the transactivation function of the AR with the exception of Bag-1ΔC that is already known not to enhance the transactivation function of the AR (Figs. 2A and 2B). When a set of mutants with deletions in the ubiquitin-like domain (Bag-1LΔUbi) and sequences between the ubiquitin-like domain and the Bag-domain (Bag-1LΔ202-224; Bag-1LΔ202-269) (Fig. 2D) were used, a different result was obtained. Two of the mutants, Bag-1LΔUbi and Bag-1LΔ202-224 bound the hormone response element of the MMTV LTR while binding by Bag-1LΔ202-269 was severely compromised (Fig. 2E). These truncated Bag-1L proteins were expressed at similar levels as shown in an immunoblot experiment where their levels were compared to the cellular levels of Hsp70 (Fig. 2D). Intriguingly, Bag-1LΔUbi and Bag-1LΔ202-224 but not Bag-1LΔ202-269 enhanced the transactivation function of the AR to a similar extent as Bag-1L (Fig. 2F). Thus, the region 202-269 of Bag-1L contributes both to binding to the HRE as well as to enhancing the AR action while the region 298-345 deleted in Bag-1LΔC contributes only to enhancing the transactivation function of the AR.

The Bag-1LΔ202-269 truncated protein that does not bind chromatin nor enhance the activity of the AR also has an impaired AR and Hsc70/Hsp70 binding. In GST pull down experiments it could be shown that while the Bag-1L and the two mutant proteins Bag-1LΔUbi and Bag-1LΔ202-224 bound Hsp70 and the AR in the absence and presence of hormone, Bag-1LΔ202-269 did not bind to these proteins (Fig. 3, A and B). This suggests that either the deleted Bag-1L sequences is important for binding Hsp70, AR and chromatin or it is an essential component in the overall structure of Bag-1L such that when deleted, it destroys the conformation of the Bag-1L protein necessary for Hsp70, AR and chromatin binding.

### Example 3:

### Characterization of the Bag-1L 202-269 peptide

To characterize the 202-269 Bag-1L peptide, a GST construct was generated and this protein bacterially purified for in vitro pull down studies. As control, the GST protein alone and GST fusion protein consisting of the peptide and the Bag-domain (amino acids 202-345) were used. These proteins were immobilized on glutathione-sepharose beads and allowed to interact with lysates from 22Rv1 and LNCaP cells. Specific binding of cellular proteins to the immobilized peptides was detected by immunoblotting.

It could be shown that Hsp70 and the AR that are known to interact with the Bag-domain bound to the 202-345 but not the 202-269 peptide (Fig. 4A, compare lanes 5 and 6 with 3 and 4). Antibodies to other proteins that are known to interact with Bag-1 such as Raf-1 and Rb were also used to demonstrate protein binding but none of these proteins bound the 202-269 peptide although they showed a weak interaction to the Bag-domain containing peptide (202-345) (Fig. 4A, compare lanes 5 and 6 with 3 and 4). No evidence for dimerization of the Bag-1 protein could be found. Lysates from Bag-1L-transfected cells did not show any interaction of Bag-1L (using a Bag-1L specific antibody) to the Bag-domain containing peptide or to the 202-269 peptide.

To determine how the 202-269 peptide could contribute to chromatin binding and to the inhibition of Bag-1L-mediated enhancement of androgen receptor action, specific proteins from 22Rv.1 and LNCaP cells that bind to the 202-269 and 202-345 peptides were determined in an unbiased manner in a GST pull-down study. Two main proteins that bound to both 202-269 and 202-345 peptides in the 75 kDa and 30 kDa range (Fig. 4B) were identified. These proteins were identified by nano-flow liquid chromatography combined with electrospray ionization tandem mass spectrometry (nanoLC-ESI-MSMS) as GRP75, GRP78, polyubiquitin, and histone H2ai. Confirmation of these interactions was started in pull-down experiments using antibodies against GRP75 (otherwise known as mortalin) and GRP78. The results obtained so far show that GRP75 binds strongly to the 202-269 Bag-1L peptide whereas GRP78 shows a weak binding (Fig. 4C). Both proteins however bind strongly to the Bag-domain containing peptide (Fig. 4C). The identification of histone H2a.i as an interacting partner for the peptide explains the involvement of this region in chromatin binding and regulation of AR action at the ARE. However the binding of this peptide to both GRP75 and GRP78, known survival factors over-expressed in tumor cells, suggests a possible interference of the Bag-1L (202-269) peptide with prostate cell growth and survival.

### Example 4:

### Overexpression of the Bag-1L 202-269 peptide in prostate cells

To examine the action of the Bag-1L 202-269 peptide on androgen action and cell survival, several stable 22Rv.1 clones were first generated by transfecting a vector expressing an HA-tagged Bag-1L (202-269) peptide to which a NLS from the SV40 has been fused. Clones expressing different levels of the peptide and empty expression vectors were isolated and analyzed (Fig. 5A).

Experiments to determine androgen regulation in these clones did not show any significant effect of the overexpression of the peptide on androgen response of the PSA or kallikrein-2 gene expression except for an upregulation of the basal level of expression. This is consistent with the finding that this peptide does not bind the androgen receptor. The Bag-1L 202-269 peptide however had an effect on cell proliferation. BrdU incorporation studies showed that clones over-expressing high levels of the peptide (e.g. clones 34, 25 and 42) proliferated significantly less than those expressing lower levels of the peptide (clones 41 and 63) or the clones with the empty expression vectors (Fig. 5B).

A further analysis of the growth inhibitory effect of the peptide was performed by the injection of the clones into the flank of athymic nude mice. The cells containing empty expression vector developed tumors with an average allowable volume of 120 cm³ in 4 to 9 weeks (Fig. 6A), while cells expressing reduced level of the peptide achieved this volume in 6-9 weeks (Fig. 6B). In contrast, cells expressing increased level of the peptide only reached a volume of 40 cm³ in 9 weeks (Fig 6C). Upon termination of the experiments, the tumors were excised and weighed (Table 1). Note that in some cases, (e.g. clone 25), the experiment was terminated earlier than 9 weeks not because of the size of the tumor but because they were necrotic (Table 1). These results together with the growth curves show not only a delay in tumor development but also a reduction in the size and weight of the tumors in cells over-expressing the Bag-1L 202-269 peptide. Immunohistological studies to determine the reason for the decrease in size of the tumors revealed massive increase in apoptosis in the cells expressing high levels of the Bag-1L peptide such as clones 42 and 25 (Fig. 6B).

**Table 1**

| | **Clone No.** | **Tumor weight (g)** | **Termination time (weeks)** |
|---|---|---|---|
| | V33 | 1.24 ± 0.61 | 4 |
| **Vector** | V19 | 1.36 ± 0.54 | 4-5 |
| **clone** | V18 | 1.73 ± 0.48 | 7-8 |
| | V7 | 1.78 ± 0.54 | 9 |
| | P41 | 1.09 ± 0.41 | 6-7 |
| **Weak peptide** | P63 | 1.27 ± 0.29 | 6-7 |
| **clones** | P34 | 0.96 ± 0.35 | 7-8 |
| | P25 | 0.31 ± 0.14 | 7-9 |
| **Strong peptide** | P42 | 0.38 ± 0.20 | 9 |
| **clones** | P29 | 0.42 ± 0.17 | 9 |

In summary, increased expression of the Bag-1L protein has been detected in different tumors. Bag-1 gene haploid insufficiency decreases prostate cancer growth and prolongs the life span of transgenic mice that develop prostate cancer under the control of the SV40 t-antigen gene. Furthermore, nuclear Bag-1L binds to chromatin and a 67 amino acid sequence relevant for this interaction was identified. Surprisingly, overexpression of this peptide in a prostate cell line strongly inhibits growth of the cells *in vitro* and in an *in vivo* mouse xenograft model of prostate cancer. The growth inhibitory action of this peptide in the xenograft model is linked to its ability to induce apoptosis. Analysis of the mechanism of action of the peptide shows that it strongly interacts with the molecular chaperones GRP75 (otherwise known as mortalin) and weakly with GRP78. As these two proteins are highly expressed in different tumors and have been implicated in tumor growth, their interaction with the Bag-1 peptide may provide mechanistically explanation for the pro-apoptotic action of the peptide. Therefore, a Bag-1 peptide which when expressed in prostate tumor and possibly other tumors would inhibit growth and survival of these tumors was identified. Thus this peptide is a candidate for broad-spectrum molecular cancer therapy.

Bag-1 gene haploid insufficiency decreases prostate cancer growth and prolongs the life span of transgenic mice that develop prostate cancer under the control of SV40 t-antigen gene. Making use of the enhancing action of Bag-1L on AR-mediated gene expression, a sequence in the Bag-1L protein was identified which when deleted inhibits Bag-1L effect on AR action. This peptide strongly inhibits prostate cancer growth *in vitro* and *in vivo* but does not itself interact with the AR, nor does it inhibit AR action making it an appropriate therapeutic approach for hormone resistant prostate cancer, and, since the peptide strongly interacts with the molecular chaperones GRP75 (otherwise known as mortalin) and weakly with GRP78, which are highly expressed in different tumors and have been implicated in tumor growth, other forms of cancer as well.

Use was made of the ability of Bag-1L to enhance AR activity even in the presence of anti-androgens to generate a dominant negative mutant of this protein that should inhibit its effect on the AR and also inhibit prostate cancer cell growth. Bag-1L is already present on chromatin at androgen response elements in the absence of hormone, and it could be shown that the destruction of this chromatin binding property impaired the enhancement of AR transactivation by Bag-1L. The sequence (amino acids 202-269) identified to be necessary for chromatin binding overlaps helix 1 of the Bag-domain at its carboxy-terminus and extends further eleven amino acid residues at the N-terminus towards the ubiquitin-like domain. There are no structural data available for this region of the Bag-1L protein nor known homologies that could reveal a possible function for this region. However, it could be shown that this peptide does not bind Hsp70 consistent with previous findings that the Hsp70 binding site is on helicies 2 and 3 of the Bag-domain. The 202-269 Bag-1L peptide also does not bind the AR, consistent with extensive mutagenesis studies of the Bag-domain that showed that the major contact site of the AR on the Bag-domain lies outside this peptide sequence at lysine 279. However, overexpression of the Bag-1L peptide in 22Rv.1 prostate cells inhibited proliferation *in vitro* and the growth of these tumor cells in athymic nude mice *in vivo*. The inhibitory effect of the peptide *in vitro* and *in vivo* was not affected by androgens, confirming that the peptide is acting independent of the AR.

The Bag-1L peptide showed a strong binding to the molecular chaperones GRP75 and a weaker binding to GRP78 but not to GRP94. Thus, the peptide interaction surface seems to be rather specific in the protein it interacts with. GRP75 and GRP78 are both molecular chaperones that play important roles in maintaining cellular homeostasis. Both proteins are over-expressed in different tumors including prostate tumor. They have a wide cellular localization including the perinuclear region, a site that would bring them into contact with our over-expressed Bag-1L peptide. In addition, GRP75 has been discovered on cell surface of cancer cells but not on normal tissue and increased expression of GRP78 is associated with castration resistance and androgen deprivation.

GRP75 in particular has been shown to promote tumor growth by interacting with the tumor suppressor protein p53 to inactivate its transcriptional activity and anti-apoptotic function. It is possible that the Bag-1L peptide interferes with this function. Whatever the mechanism by which this peptide interferes with the action of GRP75 and GRP78, a Bag-1L peptide was identified which when over-expressed would inhibit prostate cancer growth even in the absence of androgen. This peptide is therefore an important candidate for the treatment of advanced therapy resistant prostate cancer and possibly a broad range of other tumors.

## Claims

1. A polypeptide comprising the amino acid sequence having SEQ ID NO. 1, or a functionally active derivative thereof, wherein the polypeptide is not naturally occurring Bag-1.

2. The polypeptide of claim 1, consisting of SEQ ID NO.1, or a functionally active derivative thereof.

3. The polypeptide of claim 1 or 2, which is a recombinant polypeptide.

4. A nucleotide sequence encoding a polypeptide according to any of claims 1 to 3.

5. A vector comprising the nucleotide sequence according to claim 4.

6. A host cell containing the vector of claim 5.

7. The polypeptide of any one of claims 1 to 3 for use as a medicament.

8. The polypeptide of claim 7 for use in the treatment of a neoplastic condition in a patient.

9. The polypeptide of claim 8, wherein the neoplastic condition is selected from the group consisting of breast cancer, lung cancer, endometrial cancer, gastrointestinal tract cancer and prostate cancer.

10. The polypeptide of claim 9, wherein the neoplastic condition is prostate cancer.

11. The polypeptide of claim 10, wherein the prostate cancer is a hormone refractory prostate cancer.

12. A pharmaceutical composition comprising, in a therapeutically effective amount, the polypeptide of any one of claims 1 to 3, optionally in combination with one or more pharmaceutically acceptable excipient(s) and/or carrier(s) and/or diluent(s) and/or solvent(s) and/or salt(s) and/or buffer(s).

13. A method for the detection of a cell expressing at least one molecular chaperone, comprising
(a) contacting said cell with the polypeptide of any one of claims 1 to 3 under conditions suitable for the binding of said polypeptide to the molecular chaperone; and
(b) detecting said polypeptide.

14. The method of claim 13, wherein the molecular chaperone is selected from the group consisting of GRP75 and GRP78.

15. The method of anyone of claims 13 and 14, wherein said cell is a neoplastic or a cancer cell.


SEQUENCE LISTING

<110>  Forschungszentrum Karlsruhe GmbH

<120>  Bag-1 Peptide

<130>  F 2046EU

<160>  4

<170>  PatentIn version 3.5

<210>  1
<211>  68
<212>  PRT
<213>  Artificial Sequence

<220>
<223>  peptide

<400>  1

Thr Pro Leu Ser Ala Leu Gly Ile Gln Asp Gly Cys Arg Val Met Leu
1               5                   10                  15

Ile Gly Lys Lys Asn Ser Pro Gln Glu Glu Val Glu Leu Lys Lys Leu
            20                  25                  30

Lys His Leu Glu Lys Ser Val Glu Lys Ile Ala Asp Gln Leu Glu Glu
        35                  40                  45

Leu Asn Lys Glu Leu Thr Gly Ile Gln Gln Gly Phe Leu Pro Lys Asp
    50                  55                  60

Leu Gln Ala Glu
65

<210>  2
<211>  204
<212>  DNA
<213>  Artificial Sequence

<220>
<223>  nucleotide sequence

<400>  2
acaccgttgt cagcacttgg aatacaagat ggttgccggg tcatgttaat tgggaaaaag

60

aacagtccac aggaagaggt tgaactaaag aagttgaaac atttggagaa gtctgtggag
120

aagatagctg accagctgga agagttgaat aaagagctta ctggaatcca gcagggtttt
180

ctgcccaagg atttgcaagc tgaa
204

<210> 3
<211> 23
<212> DNA
<213> Artificial Sequence

<220>
<223> primer MMTV for

<400> 3
ctgcagatac ttttggcatg agc
23

<210> 4
<211> 30
<212> DNA
<213> Artificial Sequence

<220>
<223> primer MMTV rev

<400> 4
ggtttacata agcatttaca taagacttgg
30